(19) Europäisches Patentamt
European Patent Office
Office européen des brevets

(11) EP 1 533 288 A2

(12) **EUROPEAN PATENT APPLICATION**

(43) Date of publication:
**25.05.2005 Bulletin 2005/21**

(51) Int Cl.[7]: **C07C 2/66**

(21) Application number: **04256971.5**

(22) Date of filing: **10.11.2004**

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(30) Priority: **12.11.2003 RU 2003133191**
**18.12.2003 US 741324**

(71) Applicant: **GENERAL ELECTRIC COMPANY**
**Schenectady, NY 12345 (US)**

(72) Inventors:
- **Ponomareva, Olga Alexandrovna**
**Moscow 123373 (RU)**
- **Yushchenko, Valentina Victorovna**
**Moscow 117342 (RU)**
- **Kuznetsov, Andrei Sergeevich**
**B 2003 Moscow 117234 (RU)**
- **Smirnov, Andrei Valentinovich**
**Moscow 109451 (RU)**
- **Knyazeva, Elena Evgenievna**
**Moscow 113172 (RU)**
- **Ivanova, Irina Igorevna**
**Moscow 117333 (RU)**
- **Moskovskaya, Irina Fedorovna**
**Moscow 121552 (RU)**
- **Soloveichik, Grigorii Lev**
**Latham New York 12110 (US)**

(74) Representative: **Szary, Anne C. et al**
**London Patent Operation**
**General Electric International, Inc.**
**15 John Adam Street**
**London WC2N 6LU (GB)**

(54) **Continuous Process for preparing 4,4'-diisopropylbiphenyl**

(57) A continuous flow process has been discovered for the highly selective isopropylation of biphenyl to 4,4'-diisopropylbiphenyl. Thus biphenyl and propene in decalin are passed through a solid catalyst bed contained in a flow reactor at moderate temperature (220°C) and pressure (10-30 atm) together with a continuous stream of nitrogen. The catalyst is an acidic zeolite catalyst having a molar ratio of $SiO_2$ to $Al_2O_3$ in a range between about 20 to 1 and about 500 to 1. Optimal catalytic performance is achieved when 35% or more of the active sites in the catalyst have an activation energy of ammonia desorption in a range between about 145 kJ/mol and about 170 kJ/mol. Additional enhancements of catalyst performance can be achieved by pretreating the acidic zeolite catalyst with a volatile basic agent prior to the alkylation reaction.

EP 1 533 288 A2

## Description

This invention relates to a method for the continuous preparation of 4,4'-diisopropylbiphenyl using solid acidic zeolite catalysts.

Aromatic hydrocarbons having alkyl substituents are widely used in a variety of fields and those having a substituent at a para-position are especially important. For example, they are used as starting materials for polymers, as intermediates for dyes, drugs and agricultural chemicals, or as starting materials for liquid crystalline polymers. The present invention relates to a process for producing with good selectivity a compound having alkyl groups at one or both para-positions (positions numbered 4 and 4') of biphenyl. The para alkylated aromatic compound 4,4'-diisopropylbiphenyl is a versatile chemical intermediate which can be converted to readily useful monomers such as 4,4'-biphenyl dicarboxylic acid or 4,4'-dihydroxybiphenyl (4,4'-biphenol) by oxidation of side hydrocarbon chains under appropriate oxidation conditions. For example, 4,4'-biphenyl dicarboxylic acid may be produced from 4,4'-diisopropylbiphenyl under the reaction conditions of the Amoco "Mid-Century" process .

Known continuous processes for the preparation of 4,4'-diisopropylbiphenyl by alkylation of biphenyl suffer from a lack of selectivity or low yields of the target product. Thus, attempts to prepare 4,4'-diisopropylbiphenyl in a continuous process at atmospheric pressure were limited by poor conversion and low selectivity (G. Kamalakar et al., Indian J.Chem.Technol. 6, 2, 1999; D.Vergani et al., Applied Catalysis 163, 1997).

Batch type processes for the preparation of 4,4'-diisopropylbiphenyl using dealuminated zeolite catalysts demonstrate better selectivity than the known continuous processes but suffer from low productivity.

Batch methods for producing a dialkylbiphenyl such as 4,4'-diisopropylbiphenyl include (i) reacting biphenyl with an olefin or an alkyl halide in the presence of a Friedel-Crafts catalyst such as aluminum chloride (See D.B.Priddy, "Alkylation of Biphenyl Under Mild Friedel Crafts Conditions", I & EC Product Research and Development, Vol.8, No. 3, Sept. 1969), and (ii) reacting biphenyl with olefin or an alcohol in the presence of a solid acidic catalyst such as a silica-alumina zeolite (J.S. Lee et al., Catalysis Letters, 2, 1989), or other catalysts (G.Kamalakar et al., Indian J.Chem. Technol. 6, 2, 1999).

Zeolites both natural and synthetic, exhibit a wide array of catalytic properties for various chemical transformations, for example hydrocarbon isomerization, cracking, polymerization and alkylation of aromatic hydrocarbons. For some applications it is advantageous to "dealuminate" the zeolite prior to its use in order to improve its catalytic performance. Catalytic performance of a zeolite may be measured by reaction outcomes such as product selectivity, product quantity and catalyst stability. Dealumination of zeolites is known in the art. Conventional techniques for zeolite dealumination include hydrothermal treatment, mineral acid treatment and the like.

U.S. Patent No.s 5200168, 5238677, 3442795 disclose methods for preparing highly siliceous (i.e. "dealuminated") zeolites from crystalline aluminosilicates employing complexing or chelating agents such as ethylenediaminetetraacetic acid, carboxylic acids. Further, US Patent No. 5015797 and a published report (See Catalysis Letters, 50, 1998) illustrate the preparation of 4,4'-diisopropylbiphenyl with relatively high selectivity using a dealuminated Mordenite zeolite catalyst by reacting biphenyl with propene in a batch reactor at elevated pressures.

Another batch process for the preparation of 4,4'-diisopropylbiphenyl carried out in an autoclave at high temperature is presented in Catalysis Today 31, (1996). Batch processes for the preparation of 4,4'-diisopropylbiphenyl are inherently limited and hence undesirable because they suffer from low productivity, complex catalyst recovery and regeneration obstacles, and like problems.

It would be highly desirable to discover a method for the preparation of 4,4'-diisopropylbiphenyl in a continuous process which did not suffer from the disadvantages of the earlier processes. The present invention overcomes these and other limitations and provides an efficient method for the continuous preparation of 4,4'-diisopropylbiphenyl from biphenyl and propene employing a solid acid zeolite catalyst having a particular distribution of acidic sites.

## BRIEF SUMMARY OF THE INVENTION

The present invention provides a method for the continuous preparation of 4,4'-diisopropylbiphenyl, said method comprising:

(a) preparing an acidic zeolite catalyst having a molar ratio of $SiO_2$ to $Al_2O_3$ in a range between about 20 to 1 and about 500 to 1, said catalyst comprising acidic sites, at least 35% of said sites having an activation energy of ammonia desorption in a range between about 145 kJ/mol and about 170 kJ/mol;

(b) continuously contacting in a flow reactor (i) biphenyl, (ii) at least one inert solvent, (iii) propene, and (iv) an inert diluent gas, said flow reactor containing said acidic zeolite catalyst, said contacting being conducted at a pressure greater than about 1 atmosphere and at a temperature greater than 180°C; and

(c) continuously recovering an effluent steam comprising product 4,4'-diisopropylbiphenyl, inert solvent, and inert diluent gas.

In another aspect of the present invention, the acidic zeolite catalyst used for the selective production of 4,4'-diisopropylbiphenyl is chemically treated prior to its use, said catalyst being treated with a volatile basic agent during catalyst preparation.

DETAILED DESCRIPTION OF THE INVENTION

The present invention may be understood more readily by reference to the following detailed description of preferred embodiments of the invention and the examples included therein. In the following specification and the claims which follow, reference will be made to a number of terms which shall be defined to have the following meanings:

The singular forms "a", "an" and "the" include plural referents unless the context clearly dictates otherwise.

"Optional" or "optionally" means that the subsequently described event or circumstance may or may not occur, and that the description includes instances where the event occurs and instances where it does not.

As noted the present invention relates to the continuous preparation of 4,4'-diisopropylbiphenyl I,

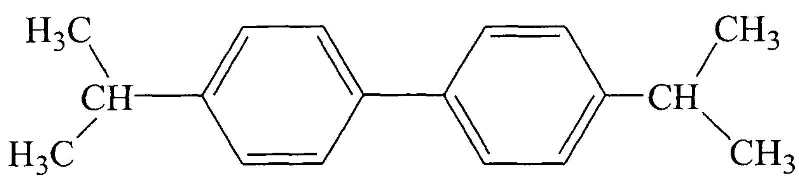

I

a valuable intermediate which may be used in the preparation of 4, 4'-biphenol II.

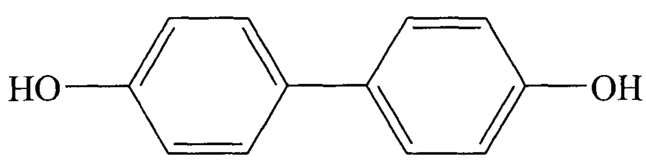

II

The present invention provides a process for selective production of 4,4'-diisopropylbiphenyl I via alkylation of biphenyl with propylene in a continuous flow reactor under elevated pressure in the presence of an acidic zeolite catalyst. Preferred catalysts include acidic zeolites prepared from precursor zeolites of the Mordenite, Mazzite, Beta types having a molar ratio of $SiO_2$ to $Al_2O_3$ structural units of from 20 to 500, and a specific distribution of acidic sites.

The solid acidic zeolite catalyst used according to the method of the present invention may be prepared from a precursor zeolite which is itself an acidic zeolite, for example zeolites of type X, Y, USY, Mazzite and Beta are available commercially and contain varying amounts of $SiO_2$ and $Al_2O_3$ structural units. In one embodiment of the present invention the precursor zeolite is Mordenite. Mordenite-type zeolites CBV 10A, CBV 21A, and CBV 90A are suitable precursor zeolites for the practice of the present invention. Mordenite-type zeolites CBV 10A, CBV 21A, and CBV 90A are available from the Zeolyst Corporation Valley Forge, PA (USA) (www.zeolyst.com).

As mentioned, the acidic zeolite catalyst used according to the method of the present invention is an acidic zeolite comprising $SiO_2$ and $Al_2O_3$ structural units, said $SiO_2$ and $Al_2O_3$ structural units being present in an amount such that the molar ratio of $SiO_2$ to $Al_2O_3$ structural units is in a range from about 20 $SiO_2$ structural units for each $Al_2O_3$ structural unit to about 500 $SiO_2$ structural units for each $Al_2O_3$ structural unit. Expressed in terms of silicon and aluminum content the molar ratio of "Si" to "Al" present in the zeolite is in a range between about 10:1 and about 250: 1. Molar ratios of silicon to aluminum in a range between about 20 to 1 and about 250 : 1 are generally preferred. Molar ratios of silicon to aluminum in a range between about 50 : 1 and about 250 : 1 are especially preferred. Acidic zeolite catalysts comprising molar ratios of silicon to aluminum in the range between about 50 to 1 and about 250 : 1 may be

prepared by the treatment of commercially available precursor zeolites as described herein.

By specific distribution of acidic sites it is meant that of the total number of acidic sites present in the zeolite, at least 35% of said sites possess an activation energy of $NH_3$ desorption in a range between about 145 kJ/mol and about 170 kJ/mol. Typically, the catalysts used according to the method of the present invention possess a distribution of acid sites characterized by 50% or more of the total number of acid sites present in the catalyst possess an activation energy of $NH_3$ desorption in a range between about 145 and about 250 kJ/mol. A preferred catalyst is a Mordenite zeolite in which between about 45 and about 60% of the total number of acidic sites possess an activation energy of $NH_3$ desorption in a range between 145 and 170 kJ/mol and between 10 and 40% of the total number of acidic sites possess an activation energy of $NH_3$ desorption greater than about 170 kJ/mol. It has been discovered that when at least 35% of the total number of acidic sites present in the catalyst possess an activation energy of $NH_3$ desorption in a range between about 145 kJ/mol and about 170 kJ/mol, the catalyst displays enhanced performance in the preparation of 4,4'-diisopropylbiphenyl.

Highly active acidic zeolite catalysts having the desired distribution of acidic sites can be prepared by modifying a precursor zeolite by either steam or thermal treatment followed by treatment with a mineral acid or an organic acid. Further improvements in catalyst selectivity can be achieved by controlled by treatment of an acidic zeolite catalyst with a volatile basic agent.

It is well known in the art that the number of tetrahedrally coordinated aluminum atoms in the zeolite framework may influence the physicochemical properties of zeolites, such as thermal stability and sorptive, ion-exchange and catalytic abilities. "Dealumination", the removal of aluminum from the zeolite framework of zeolites, is thus important from a standpoint of controlling the physicochemical properties. Dealumination is generally applied to increase hydrothermal stability and to generate mesopores, which help in overcoming diffusion barriers in the zeolite micropores. Zeolites which are relative poorer in aluminum atoms and enriched in silicon atoms have higher thermal and hydrothermal stability than do conventional zeolites. The dealumination of the zeolite framework can be carried out by hydrothermal and chemical treatments. In addition, various reagents have been used to affect chemical dealumination, for example ammonium silicon hexafluoride.

Typically, the acidic zeolite catalysts used according to the method of the present invention are prepared by subjecting a precursor zeolite to steam treatment (hydrothermal treatment) or thermal treatment in the absence of steam (calcination) at elevated temperatures followed by exposure of the steam treated or thermally treated zeolite with a mineral acid, an organic acid, or a combination thereof.

The steam treatment is carried out at a temperature in the range between about 500°C and about 800 °C. In one embodiment of the present invention, the steam treatment is carried out a range between about 700 °C and about 760 °C. The steam treatment is carried out for a duration of about thirty minutes, and alternately for about forty five minutes. It is sometimes preferred that the dealumination by steam treatment be carried for one or more hours. Typically the pressure at which the steam treatment is conducted is in a range between about 1 and about 10 atmospheres, alternatively between about 1 and about 5 atmospheres.

Thermal treatment comprises heating the zeolite undergoing dealumination in the absence of added steam. Thermal treatment may be carried out in the presence of a gas, for example oxygen, nitrogen or air. Typically, when the dealumination is carried out via thermal treatment, the zeolite undergoing dealumination is heated in the absence of exogenous steam for a period of between about thirty minutes and 10 hours. In one embodiment the thermal treatment is carried for at least forty five minutes. In an alternate embodiment the thermal treatment is carried for at least one hour. In yet another embodiment the thermal treatment is carried for at least two hours. Thermal treatment is carried out at a temperature in a range between about 500°C and about 800°C. In one embodiment of the present invention, thermal treatment is carried out in a range between about 550°C and about 800°C. In still another embodiment of the present invention, thermal treatment is carried out in a range between about 700 °C and about 760 °C. Typically the pressure at which the thermal treatment is conducted is in a range between about 1 and about 10 atmospheres, alternatively between about 1 and about 1.5 atmospheres.

Acid treatment following the steam or thermal treatment is performed by stirring the steam treated or thermally treated zeolite with an aqueous acid solution at about the reflux temperature of the aqueous acid for a period of least 0.1 hour. The preferred duration of the acid exposure of the steam treated or thermally treated zeolite may vary with the type of acid employed. For example, a period of about 0.25 hours is sufficient for aqueous sulfuric acid or aqueous nitric acid, whereas a period of about 4 hours is preferred when the acid employed is aqueous oxalic acid. Following acid exposure of the steam treated or thermally treated zeolite, the catalyst is filtered, washed with distilled water, and dried at a temperature in a range between about 80°C and about 150°C.

Acids suitable for use in the acid exposure of the steam treated or thermally treated zeolite include aqueous nitric acid ($HNO_3$), aqueous sulfuric acid ($H_2SO_4$) , aqueous hydrochloric acid (HCl), oxalic acid ($H_2C_2O_4$) and aqueous solutions prepared from ammonium nitrate ($NH_4NO_3$) and sulfuric acid. In certain instances the use of aqueous oxalic acid is preferred. The concentration of the aqueous acid employed is typically in a range between about 0.1 and about 2 molar. The preferred concentration may vary with the identity of the acid. For example 1 molar nitric acid and 0.5

molar sulfuric acid are typically preferred among the mineral acids, while 2 molar oxalic acid is typically preferred among the organic acids.

To place the various elements of acidic zeolite catalyst preparation in perspective, it should be noted that when a Mordenite zeolite is dealuminated by calcination at a temperature in a range between about 740-760°C for a period of at least 2 hours and is subsequently exposed to 2 molar oxalic acid for 1 hour at about 100°C, and thereafter is filtered washed with water and dried, the product acidic zeolite has a distribution of acid sites wherein at least 35% of the total number of acidic sites possess an activation energy of ammonia desorption in a range between about 145 kJ/mol and about 170 kJ/mol.

In one embodiment of the present invention the acidic zeolite catalyst is contacted with a volatile basic agent prior to its use as an alkylation catalyst. While not wishing to be bound by any theory, it is believed that the volatile basic agent alters catalyst performance by interaction with the acidic sites of the catalyst. The volatile basic agent is selected from the group consisting of amines, phosphines, and nitrogen-containing heterocyclic aromatic compounds and mixtures thereof. In one embodiment of the present invention the volatile basic agent is ammonia.

Typically, the acidic zeolite catalyst having a molar ratio of $SiO_2$ to $Al_2O_3$ in a range between about 20 to 1 and about 500 to 1, and at least 35% of the acidic sites having an activation energy of ammonia desorption in a range between about 145 kJ/mol and about 170 kJ/mol is treated with the volatile basic agent at a temperature in a range between about 0°C and about 500°C and at a pressure in a range between about 0.1 torr and about 10 atmospheres with at least one member selected from the group consisting of volatile amines and volatile phosphines. By "volatile" it is meant that the amine or phosphine in question has a boiling point in a range between about - 100°C and about 320°C at a pressure in a range between about 0.1 torr and about 10 atmospheres. Volatile basic agents which are amines include ammonia, alkyl amines, dialkyl amines, trialkyl amines, cycloaliphatic amines, aromatic amines, and nitrogen-containing heterocyclic aromatic compounds. Alkyl amines, dialkyl amines, trialkyl amines, and cycloaliphatic amines are exemplified by methylamine, dimethylamine, trimethylamine, ethylamine, diethylamine, triethylamine, butylamine, dibutylamine, tributylamine, decylamine, didecylanine, tridecylamine, ethylenediamine, N,N,N',N'-tetramethylethylnenediamine, piperidine, morhpholine, N-methylpiperidine, N-methylmorpholine, 1,4-diazobicyclo[2.2.2]octane (DABCO), and the like. Aromatic amines are illustrated by aniline, N-methylaniline and N,N-dimethylaniline.

Examples of volatile basic agents which are nitrogen-containing heterocyclic aromatic compounds include, imidazole, N-methylimidazole, pyridine, pyrazine, quinoline, isoquinoline, acridine, bipyridine and terpyridine.

Examples of volatile basic agents which are phosphines include phosphine ($PH_3$), alkyl phosphines, dialkyl phosphines, trialkyl phosphines, cycloaliphatic phosphines, aromatic phosphines, and the like. Alkyl phosphines, dialkyl phosphines, trialkyl phosphines, cycloaliphatic phosphines, and aromatic phosphines are illustrated by methylphosphine ($MePH_2$), dimethylphosphine ($Me_2PH$), trimethylphosphine ($Me_3P$), butylphosphine, dibutylphosphine, tributylphosphine, 1-methyl-1-phosphphacyclohexane and triphenylphosphine.

The acidic zeolite catalyst may be contacted with a volatile basic agent in a separate step as part of the catalyst preparation process, or after charging the continuous reactor used to produce 4,4-diisopropylbiphenyl with the acidic zeolite catalyst. Typically, the acidic zeolite catalyst is charged to a continuous reactor prior to contacting with a volatile basic agent.

The contacting of the acidic acid catalyst may be carried out in a manner such that the catalyst is essentially saturated with the volatile basic agent. Following saturation of the catalyst the resultant saturated acidic catalyst may be heated in the presence of a flowing inert gas (e.g. nitrogen) in order to remove a portion or essentially all of the volatile basic agent. For example, an acidic zeolite catalyst configured as a bed in a flow reactor may be contacted with ammonia by passing a stream of ammonia gas through the flow reactor at ambient temperature to provide an acidic catalyst which is saturated with ammonia. Thereafter, the flow of ammonia is discontinued and the catalyst is heated in the presence of a stream of helium at 400°C to provide an acidic catalyst in which all but the most strongly bound ammonia has been displaced from the catalyst. It is believed that the effect of this treatment is to provide a catalyst in which a portion of the most highly acidic sites present in the catalyst have been modified by the presence of the ammonia. Alternately the contacting of the acidic catalyst with a basic agent may be carried out at relatively high temperature (e.g. 500°C) in the presence of a flowing inert gas, in which instance a relatively small fraction of the acidic sites are expected to bind the volatile basic agent and be modified thereby.

The method according to the present invention is said to be "continuous", meaning that reactants biphenyl and propene, and inert components such as solvent and inert gas are continuously introduced into a flow reactor and a product stream is continuously removed from said flow reactor. Flow reactors useful according to the method of the present invention include tube reactors, trickle column reactors, continuous stirred tank reactors, fluidized bed reactors, and the like.

The flow reactor used according to the method of the present invention contains at least one solid acidic zeolite catalyst disposed within it. The solid acidic zeolite catalyst may be configured as a single fixed bed within the flow reactor, or may be configured as a series of multiple fixed beds arrayed along the length of the flow reactor, said multiple fixed beds being separated from one another by unused volume within the flow reactor or by an inert space-filling substance such as glass beads. Additionally, the solid acidic catalyst may have dispersed within it inert space filling substances, such as glass, inert ceramic materials, inert minerals and inert metals. As mentioned, the solid acidic zeolite catalyst may also be configured as a fluidized bed, a mechanically agitated bed and the like. Additionally, the solid acidic zeolite catalyst may be configured in a "doughnut shape" wherein the catalyst is distributed along the walls of the reactor while the center of the flow reactor remains open.

Reactants may be introduced into the flow reactor at a single point along the flow reactor or at multiple points along the flow reactor. Reactants biphenyl and propene may be introduced in such a manner such that they flow in opposite directions along a tube reactor, for example. Reactants and inert gas may be introduced into the flow reactor such that reactants and inert gas travel in opposite directions, for example a vertically mounted tube reactor containing a fixed or fluidized bed of the solid acidic catalyst. The tube reactor of the foregoing example has feed inlets for propene, and a solution of biphenyl in an inert solvent at the top of the reactor and an inlet for the inert gas at the bottom of the vertically mounted tube reactor. The vertically mounted tube reactor of this example is further equipped with outlets at both the top and bottom of the reactor to accommodate the outflow of products and inert gas respectively.

Regardless of the exact type of flow reactor employed or the configuration of the solid acidic zeolite catalyst within it, what is essential for the operation of the present invention is the simultaneous presence in the flow reactor of the solid acidic zeolite catalyst, the reactants (biphenyl and propene), and the inert components (solvent and inert gas), under conditions which promote product 4,4'-diisopropylbiphenyl formation. This simultaneous presence requirement is described in terms of "continuously contacting" the solid acidic catalyst with biphenyl, at least one inert solvent, propene, and an inert diluent gas.

Although not wishing to be bound by any particular theory of operation, it is believed that 4,4'-diisopropylbiphenyl is produced with the greater efficiency when an inert diluent gas is employed according to the method of the present invention, and this greater efficiency is related to an enhanced removal and recovery of the product 4,4'-diisopropylbiphenyl. As a result of its enhanced removal, the product 4,4'-diisopropylbiphenyl is believed to suffer less "overalkylation" to undesired triisopropylated products. It may be that reaction systems comprised by the method of the present invention are sufficiently complex such that no simple chemical phenomenon-based theory adequately explains the enhanced performance observed.

Typically, the reactant biphenyl is introduced into the flow reactor as a solution in an inert solvent. The choice of solvent is not particularly critical as long as the solvent is chemically inert. Typically, the inert solvent used according to the method of the present invention is an inert hydrocarbon solvent having a boiling point in a range between about 80°C and about 320°C, preferably between about 180 °C and about 320 °C. Inert solvents which may be used according to the method of the present invention include saturated hydrocarbons such as decalin, decane, dodecane, tetradecane and hexadecane.

Typically, the solution of biphenyl in an inert solvent is continuously introduced into the flow reactor at a rate such that the weight hourly space velocity (WHSV) of biphenyl with respect to solid acidic catalyst is between about 0.025 hr-1 and about 10 $hr^{-1}$. In certain preferred embodiments the weight hourly space velocity of biphenyl with respect to the solid acidic catalyst is between about 0.1 hr-1 and about 2.5 hr-1. In instances in which the biphenyl is introduced independently from the inert solvent the weight hourly space velocity of biphenyl with respect to the solid acid catalyst falls within the same range of rates of introduction, namely between about 0.025 hr-1 and about 10 $hr^{1}$.

Propene is typically introduced into the flow reactor as a gas or in a solution comprising propene, biphenyl and decalin. The propene may be introduced in pure form or as a mixture with an inert gas, for example a mixture of propene with argon. The propene may be introduced through a single or a plurality of feed inlets on the flow reactor. Typically, the feed inlet through which the propene is introduced is distinct from that used to introduce biphenyl. The propene may be introduced at any point along the flow reactor but is typically introduced at a position located above the acidic catalyst bed or below the acidic catalyst in for example a vertically mounted tubular flow reactor. In one embodiment, the acidic catalyst is disposed within a vertically mounted tube reactor, said tube reactor having a top and a bottom. In this embodiment, propene, a solution of biphenyl in an inert solvent, and an inert gas are introduced from the top of said flow reactor. An effluent stream consisting of products, starting materials and inert gas exits the bottom of the reactor.

Typically, propene is introduced into the flow reactor at a rate corresponding to between about 0.1 and about 10 moles of propene per mole of biphenyl introduced. For example, an embodiment of the present invention wherein the rate of introduction of biphenyl corresponds to 1 mole of biphenyl being introduced into the flow reactor per hour and the rate of propene introduction corresponds to 10 moles of propene being introduced into the flow reactor per hour, the rate of propene introduction is said to correspond to "10 moles of propene per mole of biphenyl" introduced and propene is said to be "present" in the flow reactor in an amount corresponding to 10 moles of propene per mole of biphenyl.. In one embodiment of the present invention propene is present in the flow reactor in an amount corresponding to between about 2 and about 5 moles of propene per mole of biphenyl.

The diluent gas may be any gas which is inert under the reaction conditions and which does not interfere with the production of 4,4'-diisopropylbiphenyl. For example the inert gas used according to the method of the present

invention may be nitrogen, helium, argon, carbon dioxide, or a mixture of two or more of these gases. Typically, nitrogen is preferred. The amount of inert gas employed according to the method of the present invention has been found to impact the yield of 4,4'-diisopropylbiphenyl. It has been found that increasing the space velocity of the inert gas relative to the biphenyl and propene reactants enhances the yield of 4,4'-diisopropylbiphenyl. In addition it has been found that increasing the space velocity of the inert gas relative to the biphenyl and propene reactants enhances the "selectivity" for 4,4'-diisopropylbiphenyl. "Selectivity" for 4,4'-diisopropylbiphenyl is defined as the number of moles of 4,4'-diisopropylbiphenyl contained in the reactor effluent expressed as a percentage of the total number of moles of diisopropylated products present in the reactor effluent. Typically, the amount of inert gas employed is in a range between about 1 and about 100 moles of inert gas per mole of biphenyl introduced into the flow reactor. In one embodiment of the present invention the amount of inert gas introduced into the flow reactor is in a range between about 5 and about 75 moles of inert gas per mole of biphenyl introduced into the flow reactor. In an alternate embodiment the amount of inert gas introduced into the flow reactor is in a range between about 10 and about 40 moles of inert gas per mole of biphenyl introduced into the flow reactor.

The method of the present invention can be carried out at any pressure but is preferably carried out at supraatmospheric pressure. In one embodiment the flow reactor is operated at a pressure in a range between about 1 and about 100 atmospheres. In an alternate embodiment the flow reactor is operated at a pressure in a range between about 1 and about 75 atmospheres. In yet another embodiment the flow reactor is operated at a pressure in a range between about 2 and about 40 atmospheres. The pressure in the flow reactor may be controlled by a variety of engineering means, such as a backpressure regulator located downstream of the point at which the reactor effluent emerges from the flow reactor.

Typically, the reactor is operated at a temperature sufficient to effect the conversion of a significant fraction of the biphenyl starting material to product 4,4'-diisopropylbiphenyl. By a "significant amount" it is meant that more than about 1 percent, preferably more than about 5 percent, and still more preferably more than about 10 percent of the biphenyl introduced into the reactor is converted to 4,4'-diisopropylbiphenyl (4,4'-DIPBP) or the intermediate 4-isopropylbiphenyl (4-IPBP). Typically, the flow reactor is operated at a temperature of between about 180°C and about 320°C. In one embodiment, the flow reactor is operated at a temperature in a range between about 200°C and about 250°C.

In instances in which the biphenyl is introduced into the flow reactor as a solution in an inert solvent it is useful to gauge the feed rate of this solution in terms of "catalyst bed volumes per hour". The rate of solution introduction expressed in bed volumes per hour corresponds to the volume of solution introduced into the flow reactor over the course of one hour divided by the volume of the solid catalyst disposed within the flow reactor. Alternatively, when the inert solvent and biphenyl are introduced separately it is still frequently useful to describe the rate of biphenyl and solvent introduction in terms of catalyst bed volumes per hour. In this instance the volumes of solvent and biphenyl separately introduced over the course of an hour are combined and divided by the volume of the solid catalyst disposed within the reactor.

Typically the rate of biphenyl and inert solvent introduction into the flow reactor is between about 0.1 and about 5 catalyst bed volumes per hour. In one embodiment biphenyl is introduced into the flow reactor as a solution in at least one inert solvent at a rate corresponding to between about 1 and about 3 bed volumes per hour.

## EXAMPLES

The following examples are set forth to provide those of ordinary skill in the art with a detailed description of how the methods claimed herein are carried out and evaluated, and are not intended to limit the scope of what the inventors regard as their invention. Unless indicated otherwise, parts are by weight, temperature is in ° C. The term "weight hourly space velocity" (WHSV) refers to the weight in grams per hour of a component introduced into the reactor per gram of the solid acidic zeolite catalyst contained within the reactor. Examples are numbered. Comparative Examples are indicated as "CE-1", "CE-2" and so forth.

## GENERAL PROCEDURE FOR PREPARATION OF DEALUMINATED ZEOLITES

A precursor zeolite, CBV 90A (Mordenite zeolite), with molar ratio of $SiO_2$ to $Al_2O_3$ 97 was obtained ZEOLYST Corporation. Dealumination was carried out by steam treatment at temperature of about 740°C for 2 hours followed by treatment with 1 molar nitric acid for a duration of 2 hours. The molar ratio of $SiO_2$ to $Al_2O_3$ in the product dealuminated acidic zeolite was 183. The distribution of acid sites in this sample was determined by thermo-programmed desorption of ammonia ("TPD $NH_3$"). 0.2 grams of the acidic zeolite catalyst having a particle size of 0.25-0.5 millimeters was placed into quartz reactor, treated in a flow of dry air (flow rate 0.5 ml/s) for 4 hours at a temperature of 550°C and then placed under nitrogen flow for 1 hour at 550°C. The catalyst was cooled to ambient temperature, and subjected to a flow of ammonia in $N_2$ carrier gas ($NH_3$:$N_2$=1:1) for 0.5 hour. Weakly bound ammonia was flushed out with helium

at 100°C, the reactor was then cooled to room temperature, and programmed heating in a flow of He at a rate of 8 degrees per minute was initiated. The rate of $NH_3$ desorption was recorded as a function of temperature and a "TPD $NH_3$" curve was thus obtained. The acidic site distribution ($\Phi(\bar{E}_i)$ - dependence of the amount of sites versus activation energies) was calculated from the thermo-programmed desorption (TPD) curve according to V.V.Yuschenko et al., Russian Journal of Phys. Chem., 1999, V.73, N4, p.646-652 which is incorporated herein by reference.

Examples of catalysts prepared according to the general procedure are shown in Table 1.

Table 1.

| Sample | Catalyst | Conditions of catalysts preparation | | | | | Amounts of acid sites with different activation energies of $NH_3$ desorption (μmol/g) | | |
|---|---|---|---|---|---|---|---|---|---|
| | | Thermal treatment | | | Acid treatment | | E<145 kJ/mol | 145<E<170 kJ/mol | E>170 kJ/mol |
| | | Type | T, °C | Time hours | Acid con. | Time, hours | | | |
| A | MOR[a] (97) | - | - | - | - | - | 260.1 | 109.5 | 163.3 |
| B | MOR (183) | ST[b] | 740 | 2 | 1M $HNO_3$ | 2 | 53.6 | 34.7 | 46.7 |
| C | MOR (330) | C | 740 | 2 | 2M $HNO_3$ | 2 | 42.0 | 45.8 | 29.2 |
| D | MOR (196) | C | 750 | 2 | 1M $HNO_3$ | 2 | 56.1 | 83.2 | 80.7 |
| E | MOR (253) | C | 750 | 2 | 2M $H_2C_2O_4$ | 2 | 12.5 | 31.9 | 9.6 |
| F | MOR (340) | C | 750 | 2 | 2M $H_2C_2O_4$ | 4 | 15.37 | 26.93 | 16.68 |
| G | MOR (420) A[c] | C | 750 | 2 | 2M $H_2C_2O_4$ | 4 | 38.25 | 29.53 | 11.20 |
| H | MOR (420) B | C | 760 | 2 | 2M $H_2C_2O_4$ | 1.5 | 16.9 | 32.1 | 19.2 |
| I | MOR (460) | C | 760 | 2 | 2M $H_2C_2O_4$ | 2 | 17.4 | 32.9 | 17.7 |
| J | MOR (238)[d] | C | 750 | 2 | 2M $H_2C_2O_4$ | 2 | n/a | n/a | n/a |

[a] "MOR" = Mordenite, CBV-90A (ZEOLYST Corp.)
[b] "ST " = steam, "C " = calcinations,
[c] dealumination procedure repeated twice,
[d] prepared from Mordenite CBV-10A (ZEOLYST Corp.) "n/a" indicates data not available.

Table 1. (continued)

| Sample | Catalyst | Conditions of catalysts preparation | | | | | Amounts of acid sites with different activation energies of $NH_3$ desorption (μmol/g) | | |
|---|---|---|---|---|---|---|---|---|---|
| | | Thermal treatment | | | Acid treatment | | E<145 kJ/mol | 145<E<170 kJ/mol | E>170 kJ/mol |
| | | Type | T, °C | Time hours | Acid con. | Time, hours | | | |
| K | MAZ[e] (28) | C | 600 | 2 | 1M $HNO_3$ | 2 | 86.0 | 41.2 | 24.3 |

GENERAL PROCEDURE FOR PRODUCING 4,4'-DIISOPROPYLBIPHENYL.

A stainless steel fixed-bed type reactor having two inlets at the top of the reactor and a single outlet for effluent at the bottom of the reactor was charged with 0.7 grams of granulated CBV 10A (Mordenite-type zeolite dealuminated as described above) having a molar ratio of $SiO_2$ to $Al_2O_3$ of about 183 and a particle size of from about 0.25 to about 0.5 mm. The reactor was operated in the vertical position. Reagents and nitrogen were introduced through the two inlets at the top of the reactor and the reactor effluent emerged through an outlet at the bottom of the reactor. A 0.8 molar (0.8M) solution of biphenyl in decalin was prepared. Propene was added to the 0.8 M biphenyl in decalin solution and the resulting solution was then fed into the reactor with a syringe metering pump at about 2 to about 3 grams of the solution per hour through one of the two reactor inlets. In the Examples, nitrogen gas was introduced through the second reactor inlet. $N_2$ was delivered using a mass flow controller at the rate of 7.3 milliliters per minute. The relative molar amounts of biphenyl (BP), propene (Pr) and nitrogen ($N_2$) being introduced were 1 mole BP per 2 moles Pr per 10 moles $N_2$. This is expressed in shorthand as "BP:Pr:$N_2$ = 1:2:10 (mole)". The reactor temperature was about 220°C as measured by a thermocouple in contact with the solid catalyst located within the reactor, the reactor pressure was about 10 atmospheres, the molar ratio of biphenyl to propylene was either about 1:2 or about 1:5, and the weight hourly space velocity (WHSV) with respect to biphenyl was about 0.5 $hr^{-1}$. It should be noted that the pressure within the reactor was controlled by a back pressure regulator at the reactor outlet. The pressure in the system was maintained at a given level by adjusting the back pressure regulator to achieve the desired reactor pressure. In this way substantial amounts of nitrogen could be introduced simultaneously with reactants and solvents without resulting in an increase in the overall pressure.

Each experiment was run continuously for a period of from about 2 to about 20 hours. After passing through the reactor and back pressure regulator, the gas and liquid phases of the effluent were separated at atmospheric pressure. The reactor effluent was analyzed by gas chromatography (GC) to determine the distribution of products and starting materials. The data for the Examples and Comparative Examples are presented in Table 2 and represent reaction behavior under steady state conditions. Steady state conditions refer to the period of stable operation of the reactor wherein the percent conversion of reactants to products was neither rising nor declining and was deemed to begin at a point in time following the initial reactor start-up. The end of the steady state period was marked by a decline in percent conversion of starting materials to products. The results are shown in Table 2.

Table 2.

| Example | Catalyst | Contribution of acid sites with activation energy (E) of $NH_3$ between 145 and 170 kJ/mol relative to the total acidic sites | Yield of 4,4'-DIPB, mol% | Selectivity to 4,4'-DIPB, % |
|---|---|---|---|---|
| CE- 1 | MAZ(28) | 27.1% | 22.5 | 47.6 |
| CE-2 | MOR(97) | 20.6% | 39.6 | 66.4 |
| CE-3 | MOR(183) | 25.7% | 22.2 | 68.0 |
| 1 | MOR(330) | 39.1% | 51.0 | 81.5 |

Table 2. (continued)

| Example | Catalyst | Contribution of acid sites with activation energy (E) of $NH_3$ between 145 and 170 kJ/mol relative to the total acidic sites | Yield of 4,4'-DIPB, mol% | Selectivity to 4,4'-DIPB, % |
|---|---|---|---|---|
| 2 | MOR(196) | 37.8% | 49.2 | 74.9 |
| 3 | MOR(253) | 59.0% | 59.3 | 84.1 |
| 4 | MOR(340) | 45.6% | 53.0 | 83.5 |
| 5 | MOR(420) A | 37.4% | 49.3 | 77.6 |
| 6 | MOR(420) B | 47.1% | 53.8 | 84.1 |
| 7 | MOR(460) | 48.4% | 55.8 | 86.3 |

Example 8. A sample of commercial MOR (97) zeolite with molar ratio of $SiO_2$ to $Al_2O_3$ 97 (CBV 10A) produced by ZEOLYST was saturated with ammonia vapor at room temperature, placed in the flow reactor and heated to 420 °C under nitrogen flow and then cooled down to room temperature. The isopropylation of biphenyl using this catalyst was carried out as described in the general procedure.

Example 9. A sample of catalyst prepared according Example 8 was heated to 450 °C in nitrogen flow and then cooled down to room temperature. The isopropylation of biphenyl using this catalyst was carried out as described in the general procedure. The results are shown in Table 3. TrIPBP refers to triisopropylbiphenyl.

Table 3.

| Example | CE-2 | 8 | 9 |
|---|---|---|---|
| Catalyst | MOR(97) | MOR(97)+$NH_3$ | MOR(97)+$NH_3$ |
| Desorption temperature of $NH_3$ °C | - | 420 | 450 |
| Conversion BP, % | 89.4 | 10.0 | 47 |
| Composition of IPBP,% | | | |
| 3-IPBP | 8.7 | 13.5 | 12 |
| 4-IPBP | 9.6 | 59.7 | 36 |
| Composition of DIPBP,% | | | |
| 3,3'-DIPBP | 1.7 | 0.0 | 1 |
| 3,4'-DIPBP | 12.0 | 11.8 | 10 |
| 4,4'-DIPBP | 66.4 | 81.6 | 86 |
| Others | 19.9 | 6.6 | 3 |
| Composition of alkylated products, mol % | | | |
| IPBP | 18.5 | 73.3 | 49 |
| DIPBP | 66.8 | 23.1 | 45 |
| TrIPBP | 5.6 | 0.0 | 1 |
| Others | 9.1 | 3.6 | 6 |
| Yield of 4.4'-DIPBP, % | 39.6 | 1.9 | 18 |
| Ratio | | | |
| 4/3 | 1.1 | 4.4 | 2.9 |
| 4,4'/(3,4'+3,3') | 4.85 | 6.80 | 8.8 |
| Total para-selectivity, % | 53.9 | 78.5 | 70.8 |

Comparative Example 4 (CE-4). The isopropylation of biphenyl was carried out as described in the general procedure except that commercial Mordenite zeolite with molar ratio of $SiO_2$ to $Al_2O_3$ 8.5 (CBV 10A) obtained from ZEOLYST was used as a catalyst. The results are shown in Table 4.

Comparative Example 5 (CE-5). The isopropylation of biphenyl was carried out as described in the general procedure except that modified Mordenite MOR(238) prepared according Example 8 with molar ratio of $SiO_2$ to $Al_2O_3$ 238 was used as a catalyst. The results are shown in Table 4.

Example 10. A sample of catalyst prepared in the same manner as catalyst Sample I was saturated with ammonia vapor at room temperature, placed in the flow reactor and heated to 450°C under nitrogen flow and then cooled down to room temperature. The isopropylation of biphenyl using this catalyst was carried out as described in the general procedure. The results are shown in Table 4. TrIPBP refers to triisopropylbiphenyl.

Table 4.

| Example | CE-4 | CE-5 | 10 |
|---|---|---|---|
| Catalyst | CVB 10A | MOR(238) | MOR(238) +$NH_3$ |
| Conversion BP, % | 64 | 90 | 86 |
| Composition of DIPBP,% | | | |
| 3,3'-DIPBP | 4 | 1 | 0.7 |
| 3,4'-DIPBP | 18 | 10 | 10 |
| 4,4'-DIPBP | 76 | 87 | 89 |
| Others | 2 | 2 | 0.3 |
| Composition of alkylated products, %-mol | | | |
| IPBP | 41 | 15 | 16 |
| DIPBP | 40 | 71 | 74 |
| TrIPBP | 4 | 4 | 2 |
| Others | 15 | 11 | 8 |
| Yield of 4.4'-DIPBP, % | 19 | 56 | 57 |
| Ratio | | | |
| 4,4'/(3,4'+3,3') | 3.45 | 7.9 | 8.3 |
| Total para-selectivity, % | 57 | 69 | 74 |

The invention has been described in detail with particular reference to preferred embodiments thereof, but it will be understood by those skilled in the art that variations and modifications can be effected within the scope of the invention.

## Claims

**1.** A method for the preparation of 4,4'-diisopropylbiphenyl, said method comprising:

(a) preparing an acidic zeolite catalyst having a molar ratio of $SiO_2$ to $Al_2O_3$ in a range between about 20 to 1 and about 500 to 1, said catalyst comprising acidic sites, 35% or more of said sites having an activation energy of ammonia desorption in a range between about 145 kJ/mol and about 170 kJ/mol;

(b) continuously contacting in a flow reactor (i) biphenyl, (ii) at least one inert solvent, (iii) propene, and (iv) an inert diluent gas, said flow reactor containing said acidic zeolite catalyst, said contacting being conducted at a pressure greater than about 1 atmosphere and at a temperature greater than 180°C; and

(c) continuously recovering an effluent steam comprising product 4,4'-diisopropylbiphenyl, inert solvent, and inert diluent gas.

2. The method according to claim 1 wherein said acidic zeolite catalyst is prepared from a precursor zeolite by a method comprising a dealumination step.

3. The method according to claim 2 wherein, said dealumination step comprises steam treatment.

4. The method according to claim 2 wherein, said dealumination step comprises thermal treatment in the absence of exogenous steam.

5. The method according to claims 3 or 4 wherein, said thermal treatment is carried out for a duration of at least about two hours at a temperature in a range between about 550°C and about 800°C.

6. The method according to claim 2 wherein said dealumination step comprises contacting with aqueous acid.

7. The method according to claim 2 wherein, said precursor zeolite is selected from the group consisting of Mordenite, Mazzite, and Beta zeolites.

8. The method according to any preceding claim wherein, the biphenyl is continuously contacted with the acidic zeolite catalyst at a weight hourly space velocity (WHSV) of between about 0.025 $hr^{-1}$ and about 10 $hr^{-1}$.

9. The method according to any preceding claim wherein said preparing an acidic zeolite catalyst comprises contacting an acidic zeolite with at least one volatile basic agent at temperature range between about 0°C and about 500°C and at a pressure in a range between about 0.1 torr and about 10 atmospheres.

10. The method according to claim 9 wherein, said volatile basic agent is selected from the group consisting of amines and phosphines.